# EUROPÄISCHE PATENTANMELDUNG

(11) **EP 0 873 981 A1**
(43) Veröffentlichungstag der Anmeldung: **28.10.1998**
(21) Anmeldenummer: 98105249.1
(22) Anmeldetag: 23.03.1998
(51) Int. Cl.: C07C 7/20

(54) **Verfahren zur Vermeidung von spontanen Zerfällen gasförmigen Acetylens**

(30) Priorität: 24.03.1997 DE 19712249
(71) Anmelder: BASF AKTIENGESELLSCHAFT, 67056 Ludwigshafen (DE)
(72) Erfinder: Lorenz, Rudolf Erich, 67069 Ludwigshafen (DE); Helfert, Herbert, 67227 Frankenthal (DE); Schmidt-Radde, Martin, 67259 Beindersheim (DE); Schildberg, Hans-Peter, 67433 Neustadt (DE); Saladin, Günter, 67069 Ludwigshafen (DE)
(74) Vertreter: Kinzebach, Werner, Dr.

(57) **Zusammenfassung**

Die vorliegende Erfindung betrifft ein Verfahren zur Vermeidung eines spontanen, von gasförmigem Acetylen oder Gasmischungen, die Acetylen enthalten, in chemischen Reaktoren, das dadurch gekennzeichnet ist, daß man zumindest die den Zerfall initiierenden Reaktorbereiche mit einem mittelviskosen, chemisch inerten Öl behandelt.

Die Erfindung betrifft weiterhin ein Verfahren zur Vinylierung H-acider Verbindungen, das von dem oben genannten Verfahren Gebrauch macht.

## Beschreibung

Die vorliegende Erfindung betrifft ein Verfahren zur Vermeidung eines spontanen Zerfalls von gasförmigem Acetylen oder Gasmischungen, die Acetylen enthalten, in chemischen Reaktoren. Die vorliegende Erfindung betrifft insbesondere ein Verfahren zur Vermeidung solcher Acetylenzerfälle, die durch chemische Zünder ausgelöst werden (chemische Zerfälle).

Es ist bekannt, daß Acetylen und Acetylen enthaltende Gasmischungen unter erhöhter Temperatur und erhöhtem Druck zur spontanen Zersetzung neigen. Die Exothermie dieser Zerfälle (226 kJ/Mol Acetylen) führt zu starken Druckanstiegen, welche eine vollständige Zerstörung der Reaktionsapparatur zur Folge haben können. Man vermutet, daß derartige Zersetzungsreaktionen durch "chemische Zünder" ausgelöst werden (chemische Zerfälle). Unter "chemischen Zündern" sind chemische Verbindungen zu verstehen, die in fester Form, z.B. als Ablagerungen in Produktionsanlagen, die Zersetzung von gasförmigem Acetylen katalysieren. Hierzu zählen neben Schwermetallsalzen, wie z.B. Kupfersalzen, insbesondere auch Basen, z.B. Oxobasen wie Hydroxide, Alkoholate, Carbonate, speziell solche mit Alkalimetallen als Gegenionen, oder Carboxylate, wie Zinkstearat (siehe hierzu auch H.P. Schildberg et al, Eighth International Symposium on Loss-Prevention and Safety Promotion in the Process Industry, Antwerpen 1995). Die als potentielle "chemische Zünder" genannten chemischen Verbindungen stellen andererseits wirksame Katalysatoren bei chemischen Umsetzungen mit Acetylen dar. Auf sie kann daher nicht verzichtet werden.

In den für die Umsetzung von Acetylen unter Druck geeigneten Apparaturen können chemische Zünder prinzipiell an folgenden Stellen auftreten: Heizschlangen des Autoklaven, Innenwandung des Autoklaven, Acetyleneingangsstutzen, sonstige Stutzen zur Einleitung oder Abführung von Gasen. Während das Auftreten von chemischen Zündern an den beiden erstgenannten Stellen bereits durch einfachere Maßnahmen, wie Erhöhung der Rührerdrehzahl, vermieden werden können, besteht jedoch bei den anderen Stellen die Gefahr, daß das in der Regel flüssige Reaktionsgemisch, welches die chemischen Zünder in gelöster oder suspendierter Form enthält, dorthin spritzt, antrocknet und so chemische Zünder in aktiver Form entstehen.

Die Ausbildung derartiger aktiver chemischer Zünder kann im Prinzip auf unterschiedliche Weise verhindert werden. So ist es denkbar, die besagten Stellen mit einer Flüssigkeit, beispielsweise mit einem Lösungsmittel und/oder einem für die Umsetzung verwendeten Edukt, sofern flüssig, zu spülen. Hierbei entstehen jedoch Zusatzkosten, wie z.B. für zusätzlich verbrauchtes Lösungsmittel. Im Falle einer Eduktspülung kann diese nur über einen bestimmten Zeitraum erfolgen, da sonst kein Vollumsatz erreicht wird. Zudem verlängert sich die Batch-Zeit in nicht wünschenswerter Weise. Beide Varianten haben den Nachteil, daß zusätzliche Flüssigkeitsmengen gehandhabt werden müssen. Zudem erfordern beide Varianten eine zusätzliche Schnittstelle zum Hochdrucksystem der Anlage, was zusätzliche Kosten verursacht. Alternativ hierzu ist die Spülung besagter Stellen mit der flüssigen Reaktionsmischung denkbar. Auch bei dieser Variante ist die Handhabung größerer Flüssigkeitsmengen von Nachteil.

Die Verdünnung von Acetylen mit inerten Gasen wie z. B. Stickstoff bewirkt keine ausreichende Verminderung der Zerfallshäufigkeit. Zudem wird durch diese Maßnahme in der Regel die Umsatzdauer in nicht erwünschter Weise verlängert und der Umsatz verringert.

Aufgabe der vorliegenden Erfindung ist somit die Bereitstellung eines Verfahrens zur Vermeidung eines spontanen Zerfalls von gasförmigem Acetylen in chemischen Reaktoren, das die obigen Nachteile nicht aufweist.

Es wurde nun überraschenderweise gefunden, daß die spontane Zersetzung von gasförmigem Acetylen verhindert werden kann, wenn man diejenigen Stellen eines für die Acetylenumsetzung verwendeten Reaktors, an denen chemische Zünder auftreten können, mit einem geeigneten, chemisch inerten Öl behandelt.

Die vorliegende Erfindung betrifft somit ein Verfahren zur Vermeidung eines spontanen Zerfalls von gasförmigem Acetylen oder Gasmischungen, die Acetylen enthalten, in chemischen Reaktoren, das dadurch gekennzeichnet ist, daß man zumindest die den Zerfall initiierenden Reaktorbereiche mit einem mittelviskosen, chemisch inerten Öl behandelt.

Die erfindungsgemäße Behandlung der relevanten Reaktorbereiche, insbesondere der Stellen, an denen Ablagerungen "chemischer Zünder" auftreten können, erfolgt Vorzugsweise in Form eines Ölnebels, den man diskontinuierlich oder insbesondere kontinuierlich dem Reaktor zudosiert. Insbesondere wird der Ölnebel zusammen mit dem gasförmigen Acetylen zudosiert.

Die Zerstäubung des Öls zu einem Ölnebel geschieht nach üblichen Methoden. Eine übliche Anordnung zur Erzeugung von Ölnebeln umfasst z. B. eine Dosierpumpe und Düse, z. B. eine Hohlkegeldüse mit Drallkörper, die an den kritischen Stellen angebracht wird, z.B. im Bereich der Acetylengaszufuhr. Um eine wirkungsvolle Zerstäubung zu erreichen, erweist es sich als vorteilhaft, das Öl anzuwärmen, beispielsweise auf Temperaturen im Bereich von 80 bis 180°C, vorzugsweise im Bereich von 100 bis 150°C. Hierbei ist zu beachten, daß eine wirkungsvolle Zerstäubung des Öls nur dann gewährleistet ist, wenn das Öl im jeweiligen Temperaturbereich eine geeignete Viskosität aufweist. Bevorzugt werden daher Öle, die bei etwa 100°C eine Viskosität im Bereich von 1 bis 100 mm²/sek⁻¹, vorzugsweise 2 bis 80 mm²/sek⁻¹ und insbesondere 5 bis 50 mm²/sek⁻¹ (nach DIN 51562) aufweisen.

Die für eine effektive Inertisierung der chemischen Zünder benötigte Ölmenge liegt, je nach Bauart des Reaktors, im Bereich von 1 bis 100 ml, vorzugsweise 2 bis 80 ml, insbesondere 5 bis 50 ml Öl pro kg Acetylen und Stunde.

Weiterhin ist es erforderlich, daß das Öl unter den gegebenen Reaktionsbedingungen chemisch inert ist. Aus diesem Grunde werden die verwendeten Öle vorzugsweise ausgewählt unter Paraffinölen und/oder synthetischen Ölen, wie Polyolefinölen, z.B. Polyethylenölen, Esterölen, wasserunlöslichen Polyglykolen oder Silikonölen. Die genannten Öle sind dem Fachmann bekannt und in der Literatur ausführlich beschrieben, beispielsweise in Ullmann's Enzyklopädie der technischen Chemie, 3. Aufl., Bd. 6, S. 718ff und Bd. 15, S. 278ff. Besonders bevorzugt werden Paraffinöle, insbesondere solche, die durch Solvent-Raffination gewonnen werden. Derartige Öle sind im Handel erhältlich, beispielsweise als Kompressorenöle.

Das erfindungsgemäße Verfahren zur Vermeidung spontaner Acetylenzerfälle, speziell solcher, die durch "chemische Zünder" ausgelöst werden (chemische Zerfälle), hat sich insbesondere bei der Vinylierung organischer, H-acider Verbindungen durch Umsetzung mit Acetylen oder Acetylen enthaltenden Gasen bei erhöhtem Druck und erhöhter Temperatur in Gegenwart einer Base bewährt. Demnach betrifft die vorliegende Erfinung auch ein Verfahren zur Vinylierung H-acider, organischer Verbindungen durch Umsetzung dieser Verbindungen mit Acetylen oder Acetylen enthaltenden Gasen unter erhöhtem Druck und erhöhter Temperatur in Gegenwart einer Base, wobei man zur Vermeidung der spontanen chemischen Acetylenzerfälle oben beschriebene Verfahrensmaßnahmen anwendet.

Unter H-aciden Verbindungen sind solche Verbindungen zu verstehen, in denen wenigstens ein Wasserstoffatom an ein elektronegatives Heteroatom, z.B. Sauerstoff oder Stickstoff, gebunden ist. Geeignete H-acide Verbindungen umfassen beispielsweise lineare oder verzweigte Alkanole mit 1 bis 30 C-Atomen, wie Methanol, Ethanol, n-Propanol, i-Propanol, n-Butanol, Isobutanol, tert.-Butanol, n-Pentanol, Neopentylalkohol, tert.-Amylalkohol, 1-Hexanol, 2-Ethylhexan-1-ol, 2-Propylheptan-1-ol, n-Octanol, n-Decanol, n-Dodecanol, n-Tetradecanol, Stearylalkohol, Hydroxymethylcyclohexan, Cycloalkanole, wie Cyclopentanol oder Cyclohexanol, Diole, wie Ethylenglykol, Diethylenglykol, Triethylenglykol, Tetraethylenglykol oder höhere Polyethylenglykole mit Molmassen im Bereich von 200 bis 500, 1,4-Bis(hydroxymethyl)cyclohexan, 1,6-Hexandiol, 1,2- oder 1,3-Propandiol, 1,2-, 1,3- oder 1,4-Butandiol, Poly-(1,4-butandiol) mit Molmassen im Bereich von 150 bis 500, die Mono-C₁-C₄-alkylether der genannten Diole, Aminoalkohole wie 3-Aminopropan-1-ol, 4-Aminobutan-1-ol sowie deren Mono- oder Di-C₁-C₄-alkylderivate z. B. Mono- oder Dimethylaminopropan-1-ol, Mono- oder Diethylaminopropan-1-ol, oder Polyole wie Glycerin, Trimethylolpropan oder Pentaerythrit. Zu den H-aciden Verbindungen zählen weiterhin NH- bzw. NH₂-Gruppen enthaltende Verbindungen, vorzugsweise solche, in denen die Acidität des NH-Wasserstoffatoms erhöht ist, beispielsweise lineare, verzweigte oder cyclische Amide (Lactame), z. B. die Amide von aliphatischen C₁-C₄-Carbonsäuren wie Formamid, Acetamid etc., Pyrrolidon oder Caprolactam, Harnstoff und seine Derivate, wie N-Alkyl- oder N,N'-Dialkylharnstoffe, Imidazolidone oder Stickstoffheteroaromaten, wie Imidazol, Pyrazol, Carbazol und substituierte und/oder teilhydrierte Derivate davon, wie 2-Methylimidazolin. Weiterhin zählen hierzu auch C-H-acide Verbindungen, wie Malonsäuredialkylester und deren Derivate, z.B. Malonsäuredimethylester oder -diethylester, α-Methylbutylmalonsäuredieethylester, Cyanessigsäureester, z. B. Cyanessigmethylester oder -ethylester und Malodinitril. Bevorzugt werden OH-Gruppen enthaltende Verbindungen.

Die Umsetzung der genannten Verbindungen erfolgt vorzugsweise in Gegenwart von Oxobasen. Hierunter sind Hydroxide, Alkoholate wie Methanolat, Ethanolat, n- oder iso-Propanolat, n-, 2- oder tert.-Butanolat, Carbonate oder Carboxylate wie Acetat oder Stearat zu verstehen. Insbesondere werden Alkalimetallhydroxide oder Alkalimetallalkoholate, wie Kaliumhydroxid oder Kaliummethanolat als Oxobase verwendet. Die Umsetzung kann in Substanz oder in einem vorzugsweise polar-aprotischen Lösungsmittel wie einem cyclischen Ether z.B. Tetrahydrofuran oder einem Amid z. B. Dimethylformamid, Dimethylacetamid, N-Methylpyrrolidon durchgeführt werden.

Die Umsetzungen erfordern die Anwendung von Acetylenpartialdrücken im Bereich von 2 bis 50 bar vorzugsweise 10 bis 30 bar und Temperaturen im Bereich von 50 bis 200°C vorzugsweise 100 bis 180 °C. Unter diesen Bedingungen erweisen sich die genannten Oxobasen, insbesondere Kaliumhydroxid, als besonders wirkungsvolle chemische Zünder (siehe H. Schildberg et al). Das zur Inertisierung verwendete Öl wird vorzugsweise über die Acetylenzufuhr eingebracht. Eine optimale Ölmenge liegt bei etwa 1 bis 5 l/h bei einer Gesamtansatzgröße von 1 to.

Beispiele für Produkte, die auf diesem Wege erhältlich sind, umfassen die Monovinylether der oben genannten Alkohole, z. B. Ethylvinylether, n-Propylvinylether, Isopropylvinylether, n-Butylvinylether, Isobutylvinylether, tert.-Butylvinylether, tert.-Amylvinylether, Ethylhexylvinylether, Dodecylvinylether, Octadecylvinylether, Ethylenglykolmethylvinylether, Ethylenglykolbutylvinylether, Cyclohexylvinylether, Di-, Tri- oder Tetraethylenglykolmethylvinylether, die Mono- und Divinylether der oben genannten Diole wie Ethylenglykolmonovinylether und -divinylether, 1,4-Butandiolmonovinylether und -divinylether, 1,6-Hexandiolmonovinylether und -divinylether 1,4-Bis(hydroxymethyl)cyclohexanmonovinylether und -divinylether, Di-, Tri- und Tetraethylenglykolamonovinylether und -divinylether, Poly-(1,4-butylenglykol)monovinylether und -divinylether, weiterhin 3-Aminopropan-1-olvinylether oder 3-Diethylaminopropanolvinylether, 1-Vinylimidazol (aus Imidazol), 1-Vinylimidazolin (aus Imidazolin), 1-Vinyl-2-methylimidazolin (aus 2-Methylimidazolin), N,N'-Divinylethylenharnstoff (aus Harnstoff), N-Vinylformamid (aus Formamid), N-Vinylacetamid (aus Acetamid), N-Methyl-N-vinylacetamid (aus N-Methylacetamid), N-Vinylpyrrolidon (aus Pyrrolidon), N-Vinyl-2-piperidon (aus 2-Piperidon) und N-Vinylcaprolactam (aus ε-Caprolactam).

Eine wirkungsvolle Unterdrückung spontaner Acetylenzerfälle ist bei derartigen Reaktionen bereits dann gewährleistet, wenn in der Acetylenzuleitung, vorzugsweise im Eintrittsbereich der Acetylenzuleitung in das Reaktionsgefäß, der erfindungsgemäße Ölnebel erzeugt wird.

Das erfindungsgemäße Verfahren führt somit insbesondere bei diesen Reaktionen zu einer wirkungsvollen Vermeidung von spontanen Acetylenzerfällen.

### Beispiel: Bestimmung der Zerfallsneigung von gasförmigem Acetylen in Gegenwart von KOH als chemischem Zünder

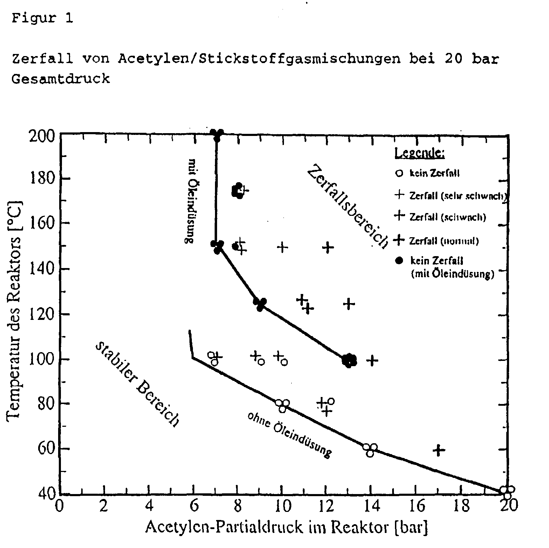

Die Erfindung wird nun unter Bezugnahme auf beiliegende Figur 1 näher erläutert. Als Öl wurde wurde das Kompressorenöl Wiol® CT 220 der Fa. Wintershall verwendet (Mineralöl; Dichte bei 15°C nach DIN 51757: 0,894 g/cm³; Viskosität bei 100°C nach DIN 51562: 17,6 mm²/s; Viskositätsindex nach DIN ISO 2909: 85; Brechungsindex nach DIN 51423: n_{D}²⁰= 1,490; Anilinpunkt nach DIN 51775: 117°C). Die Zudosiereinrichtung bestand aus einem Ölbehälter einer Kolben-Dosierpumpe, einer Hohlkegeldüse mit Drallkörper und einem Bohrungsdurchmesser von 0,1 mm. Die Düse wurde unmittelbar vor dem Hochdruckventil am Acetylenstutzen angebracht. Die Untersuchung des Acetylenzerfalls wurde an einem rußfreien 2,5 l Autoklaven durchgeführt. Hierzu gab man 4 g Kaliumhydroxid in einem Porzellanschälchen in den Autoklaven, spülte mit Stickstoff und gab dann eine Acetylen/Stickstoffmischung auf den Autoklaven, bis zu einem Gesamtdruck von 20 bar. Anschließend erwärmte man den Autoklaven auf Temperaturen oberhalb 200 °C. Es wurde notiert, ob und wie heftig ein Zerfall stattfand. Die Versuche wurden sowohl ohne als auch mit Öleinspritzung (etwa 5 ml/h) durchgeführt. Das Öl wurde vor der Eindüsung auf 120°C erwärmt.

Die Ergebnisse sind in Figur 1 zusammengefaßt: Fig. 1 zeigt in Form eines Druck-Temperaturdiagramms die Häufigkeit und Heftigkeit von Acetylenzerfällen in Stickstoff/Acetylen-Gasmischungen in Anwesenheit von KOH bei 20 bar. Dabei stehen Kreise für Druck/Temperatur-Werte, bei denen auch bei Abwesenheit einer Öleinspritzung keine Zersetzungsreaktion beobachtet wurde. Kreuze stehen für Druck/Temperaturwerte, bei denen ein spontaner Acetylenzerfall stattfand. Bei erfindungsgemäßer Anwendung eines Ölnebels kam es bei Druck/Temperatur-Verhältnissen, bei denen bei Abwesenheit der erfindungsgemäßen Maßnahme Zerfälle stattfanden, nicht mehr zu Zerfällen (gefüllte Kreise).

## Patentansprüche

1. Verfahren zur Vermeidung eines spontanen Zerfalls von gasförmigem Acetylen oder Gasmischungen, die Acetylen enthalten, in chemischen Reaktoren, dadurch gekennzeichnet, daß man zumindest die den Zerfall initiierenden Reaktorbereiche mit einem mittelviskosen, chemisch inerten Öl behandelt.

2. Verfahren nach Anspruch 1, dadurch gekennzeichnet, daß man das Öl unter Bildung eines Ölnebels dem Reaktor kontinuierlich oder diskontinuierlich zudosiert.

3. Verfahren nach einem der vorhergehenden Ansprüche, dadurch gekennzeichnet, daß man das Öl zusammen mit gasförmigem Acetylen zudosiert.

4. Verfahren nach einem der vorhergehenden Ansprüche, dadurch gekennzeichnet, daß man 1 bis 100 ml Öl pro Stunde und kg Acetylen zudosiert.

5. Verfahren nach einem der vorhergehenden Ansprüche, dadurch gekennzeichnet, daß das mittelviskose, chemisch inerte Öl bei 100°C eine Viskosität im Bereich von 1 bis 100 mm²/sek⁻¹ (nach DIN 51562) aufweist.

6. Verfahren nach einem der vorhergehenden Ansprüche, dadurch gekennzeichnet, daß es sich bei dem mittelviskosen, chemisch inerten Öl um ein Paraffinöl handelt.

7. Verfahren nach einem der vorhergehenden Ansprüche, dadurch gekennzeichnet, daß sich das gasförmige Acetylen bei einem Partialdruck oberhalb 2 bar und einer Temperatur oberhalb 50°C befindet.

8. Verfahren zur Vinylierung H-acider, organischer Verbindungen durch Umsetzung dieser Verbindungen mit Acetylen oder Acetylen enthaltenden Gasen unter erhöhtem Druck und erhöhter Temperatur in Gegenwart einer Base, dadurch gekennzeichnet, daß man zur Vermeidung spontaner Acetylenzerfälle eine Verfahrensmaßnahme aus einem der vorherigen Ansprüche ergreift.

9. Verfahren nach Anspruch 8, dadurch gekennzeichnet, daß es sich bei der Base um eine Oxobase handelt.

10. Verfahren nach einem der Ansprüche 8 oder 9, dadurch gekennzeichnet, daß die H-acide Verbindung ausgewählt ist unter linearen oder verzweigten Alkanolen, Cycloalkanolen, Diolen, Aminoalkoholen, Carbonsäureamiden, Lactamen, Harnstoff und seinen N-Alkylderivaten, Imidazolidonen und Stickstoffheteroaromaten.
